# FASCICULE DE BREVET EUROPEEN

(11) **EP 1 305 317 B1**
(45) Date de publication et mention de la délivrance du brevet: **15.09.2004**
(21) Numéro de dépôt: 01960841.3
(22) Date de dépôt: 27.07.2001
(51) Int. Cl.: C07D 487/22, A61K 31/409, A61P 35/00, A61K 49/00

(54) **DERIVES DE DIHYDROPORPHYRINE ET LEURS APPLICATIONS**
DIHYDROPORPHYRINDERIVATE UND IHRE VERWENDUNG
DIHYDROPORPHYRIN DERIVATIVES AND THEIR USES

(30) Priorité: 01.08.2000 FR 0010165
(43) Date de publication de la demande: 02.05.2003
(73) Titulaire: Anulm Association Nataise pour l'Utilisation de la Lumière en Medicine, 44093 Nantes Cedex 01 (FR)
(72) Inventeur: BOURRE, Ludovic, F-44470 Nannes sur Loire (FR); PATRICE, Thierry, F-44800 Saint Herblain (FR)
(74) Mandataire: Laget, Jean-Loup
(86) Numéro de dépôt international: PCT/FR2001/002470
(87) Numéro de publication internationale: WO 2002/010173

(56) Documents cités:
- MEHTA, GOVERDHAN ET AL: "Cholate-interspersed porphyrin-anthraquinone conjugates: Photonuclease activity of large sized, 'tweezer-like' molecules" J. CHEM. SOC., PERKIN TRANS. 1 (1999), (15), 2177-2181, XP002164715
- Dyes and Pigments 52 (2002), 199-208

## Description

L'invention concerne de nouveaux composés dérivés de dihydroporphyrine et leurs applications, notamment dans les domaines de la photochimiothérapie, en particulier pour le traitement de cancer, de l'imagerie par fluorescence et de l'optoélectronique.

Les porphyrines et leurs dérivés ont fait l'objet de nombreuses études ces dernières années en raison de leur large domaine d'application potentiel. Ainsi, les brevets US-4.992.257, US-4.837.221, US-5.162.519 et US-5.703.230 illustrent de tels exemples de composés.

Parmi ces nombreux domaines d'application, la photochimiothérapie est apparue aujourd'hui comme l'un des domaines les plus prometteurs. La photochimiothérapie est une technique de traitement qui s'est développée il y a quelques années, en particulier pour le traitement de cancers. Cette technique comprend l'administration d'un agent photosensible, à priori peu toxique, qui va être retenu avec une sélectivité relative par les tissus à index mitotique élevé, en particulier le tissu néoplasique. Cet agent photosensible est alors excité par une radiation lumineuse de longueur d'onde adaptée au spectre d'absorption lumineuse du photosensibilisant. Les radiations lumineuses absorbées par la molécule photosensible entraînent des réactions de type 1 (production de radicaux hydroxyles) ou de type 2 (production d'oxygène singulet) par un mécanisme de conversion intersystème. Ces espèces radicalaires entraînent des réactions d'oxydation et de peroxydation au niveau des tissus qui ont fixé le photosensibilisant et par suite la mort cellulaire.

L'efficacité de la technique repose sur des propriétés déterminées de l'agent photosensible. Ainsi, pour provoquer l'effet phototoxique désiré en profondeur à l'intérieur d'un tissu d'un sujet, il est nécessaire d'utiliser des photosensibilisants qui possèdent des coefficients d'absorption élevés à des longueurs d'onde supérieures à 650 nm, là où les tissus corporels sont les plus transparents à la lumière (voir Sternberg et al. "An Overview of Second Generation Drugs for Photodynamic Therapy including BPD-MA (Benzoporphyrin Derivative) *Photodynamic Therapy and Biomedical Lasers,* 470-4 *(Spinelli et al*. *eds. 1992)*.

De même, l'agent photosensible doit d'une part présenter une vitesse de pénétration rapide dans les cellules cibles à détruire de manière à réduire le temps nécessaire entre l'administration de l'agent photosensible et les radiations lumineuses, d'autre part un temps de rétention court dans les zones saines pour réduire les effets secondaires et le temps d'hospitalisation ainsi que les risques de brûlures iatrogènes lors d'exposition intempestive à la lumière. On est donc constamment à la recherche de nouvelles molécules photosensibles présentant un temps de rétention plus court dans les zones cibles de l'organisme d'un être vivant, cette durée de vie plus courte pouvant présenter un intérêt dans les autres domaines d'application, en particulier l'imagerie médicale et l'optoélectronique.

C'est pour satisfaire à ces exigences de captation rapide par le tissus cible et d'élimination rapide par les autres tissus, en comparaison des composés existants que la molécule objet de l'invention a été synthétisée.

A cet effet, l'invention a pour objet un nouveau composé de dihydroporphyrine répondant à la formule générale (I) : 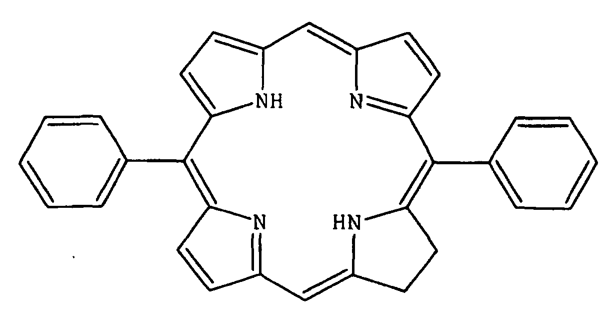 dans laquelle les groupes phényles ne sont pas substitués ou à la formule générale (II) : 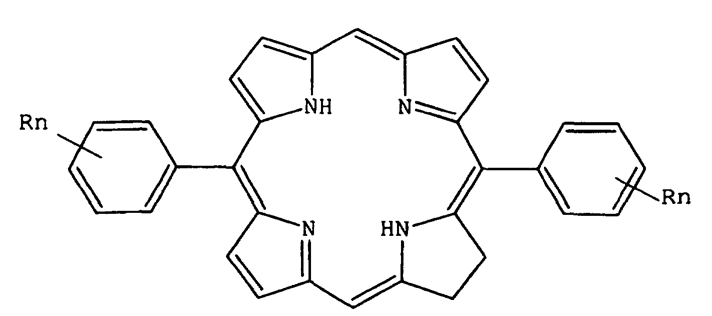 dans laquelle le groupe phényle est substitué, n est égale de 1 à 5, de préférence égale de 1 à 3, et chaque substituant R, qui peut être identique ou différent, et dans des positions identiques ou différentes sur son groupe phényle substitué est un groupe hydroxy (-OH), amine (-NH₂), sulphydrile (-SH), phosphonate (PO₃H₂, PO₃Na₂), éthylphosphonate (PO₃Et₂), sulfonate, alkyl substitué ou non, cycloalkyl substitué ou non, aliphatique, acide aminé, peptide ou polypeptide, pyridine avec différentes positions pour l'atome d'azote, purine, pyrimidine, nucléoside, saccharide, polysaccharide, acide carboxylique, un groupement amide, un groupement ester, un ammonium quaternaire substitué ou non
ou à l'un de leurs sels
ou à l'un de leurs complexes métalliques.

Les composés de l'invention peuvent exister comme mentionné ci-dessus à l'état de sel d'addition à centre acide ou basique ou à l'état de complexe métallique.

En effet, les composés décrits ci-dessus peuvent être sous la forme de dérivés tels que des sels d'addition à centre acide ou basique, des complexes métalliques, par exemple Zn, Ga, Pa, ou des hydrates ou d'autres solvates, en particulier avec des alcools aliphatiques inférieurs en C₁-C₆.

Par groupe aliphatique ci-dessus, on entend en particulier un ou plusieurs acides aminés ne comprenant pas de cycle, à savoir, par exemple, la serine ou une chaine polyéthylène glycol (PEG) ou tout autre substituant.

L'invention sera bien comprise à la lecture de la description suivante d'exemples de réalisation, en référence aux dessins annexés dans lesquels :
la figure 1 représente la toxicité et la phototoxicité de SIMO1 sur des cellules C6 laissées à incuber pendant 5 heures ;
la figure 2 représente la toxicité et la phototoxicité de SIMO1 sur des cellules C6 laissées à incuber pendant 20 heures ;
la figure 3 représente, sous forme de courbes, l'intensité de fluorescence intracellulaire des molécules SIMO1 et m-THPC ;
la figure 4 représente, sous forme de courbes, la croissance d'une tumeur mesurée à différents intervalles de temps après injection de SIMO1 ;
la figure 5 représente des courbes analogues à la figure 4 après injection de SIMO1 ou de m-THPC et
la figure 6 représente des courbes de mesure spectrofluorimétriques sur différents tissus de souris greffées à différents intervalles de temps après injection de m-THPC ou SIMO1.

L'exemple qui suit illustre la préparation d'un composé préféré de l'invention.

La synthèse de ce composé, répondant à la formule générale (III), 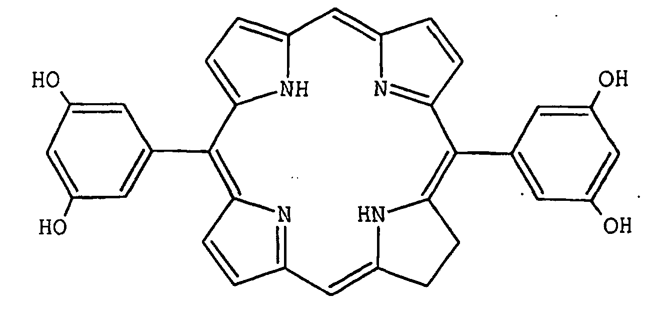 et qui sera appelé par la suite pour simplifier SIMO1 comporte les étapes suivantes.

### Etape 1 : dipyrrylméthane

Du dipyrrylméthane a été préparé comme précédemment mentionné dans Wang et Bruce, Synlett, 1267, 1995.

### Etape 2 : 5,15-Bis(3,5-diméthoxy-1-phényl)porphyrine

Du di-méthoxy-3,5-benzaldéhyde (341 mg, 2,05 mmole), du dipyrrylméthane (300 mg, 2,05 mmole) ont été ajoutés dans 205 ml de dichlorométhane distillé dans un flacon à fond circulaire. La solution a été soumise aux ultrasons dans un flux d'azote pendant 20 minutes. Ensuite, de l'acide trifluoroacétique (47 ml, 615 mmole) a été injecté et le mélange a été agité à température ambiante toute la nuit.

Une solution de DDQ 2,3-Dichloro-5,6-dicyano-1,4-benzoquinone (0,931 g dans 10 ml de toluène) a été ajoutée et le mélange de réaction a été mis sous reflux pendant 30 minutes avec un bain d'huile préchauffé à 60°C.

La purification de la porphyrine a été accomplie par chromatographie flash éluée avec de l'hexane/CH₂Cl₂ : 5/5 et a fourni 253 mg de la porphyrine désirée (36 %).

RMN 1H (CF3COOD) ppm : 11:13 (H meso), 9,7 et 9,3 (2H, d, J = 6Hz, pyrrole), 7,9 (o-H, phényl), 7,4 (p-phényl), 4,2 (méthoxy)

UV-Vis (CH₂Cl₂), max (nm) : 407, 502, 536, 574, 629.

FAB-MS : calculé pour C36H30N4O4 : 582,2 trouvé : 583 (M+H+).

### Etape 3 : 5,15-Bis(3,5-dihydroxy-1-phényl)porphyrine

Le composé de l'étape 2 (90 mg, 0,15 mmole) a été dissous dans du CH₂Cl₂ sec (10 cm³) à -20°C, suivi par l'addition de BBr₃ (0,12 cm³, 1,24 mmole). La solution verte résultante a été agitée pendant 12 heures puis placée dans de l'eau glacée. Du méthanol (20 cm³) et ensuite de l'acétate d'éthyle (10 cm³) ont été ajoutés à la suspension et le mélange a été neutralisé avec du NaHCO₃. La couche organique a été séparée, lavée d'abord avec une solution de NH₄Cl puis deux fois avec de l'eau et séchée sur du sulfate de sodium anhydre. La solution résultante a été évaporée sur un évaporateur rotatif et le résidu redissous dans de l'acétone. Après addition de pentane, une précipitation a fourni le composé de l'étape 3 avec un rendement de 52 %.

RMN 1H (acétone d6) : 10.55 (H meso), 9,6 et 9,3 (2H, d, J = 6Hz, pyrrole), 7,3 (o-H, phényl), 6,9 (p-phényl), -3,1 (NH).

UV-Vis (acétone), max (nm) : 402, 532, 572, 628.

### Etape 4 : 2,3-dihydro-5,15-Bis(3,5-dihydroxy-1-phényl)-prophyrine (SIM01)

Le composé de l'étape 3 (42 mg, 0,08 mmole), du K₂CO₃ anhydre (0,32 g) et de la pyridine anhydre (5 cm³) ont été chauffés (120°C) pendant 5 minutes. Ensuite, de faibles quantités (0,25 cm³) d'un mélange de p-toluènesulfonylhydrazide (0,44 g) dans de la pyridine (2,5 cm³) ont été ajoutés toutes les 15 mn pendant 2,5 heures. Après refroidissement, la solution de pyridine a été évaporée sous vide. La poudre résultante a été redissoute dans un mélange 1/1 d'acétone/acétate d'éthyle, puis lavée deux fois avec de l'eau et ensuite saturée avec une solution de bicarbonate. De l'o-chloranil a été ajouté en portions à la solution organique à température ambiante jusqu'à ce que le pic d'absorption à 735 nm ait disparu. La solution a été lavée deux fois avec NaHSO₄ (5 %), de l'eau distillée, NaOH (0,1 M) et du bicarbonate saturé. Après séchage sur MgSO₄, le solvant a été évaporé sur un évaporateur rotatif. Le résidu a été cristallisé à partir d'acétone/pentane pour fournir 31 mg du composé de l'étape 4 (rendement 74 %) qui sera appelé ci-après SIMO1.

RMN 1H (acétone, d6) ppm : 10,1 et 9,2 (2H-meso), 9,35-8,6 (6H, m, pyrrole), 7,2 et 6,95 (4H, d, o-phényl), 6,85 et 6,75 (2H, t, p-phényl), 4,65 et 4,45 (4H, m, pyrrolidine), - 1,4 et -1,9 (2H, s, NH).

UV-Vis (acétone), max (nm) : 395, 405, 500, 645.

La méthode de préparation de ces composés est différente de celle utilisé pour m-THPC (Brevet US 5,162,519). Elle implique une réaction entre un aldéhyde aromatique et du dipyrrylméthane ce qui aboutit à la première étape de la synthèse de SIM01 alors que m-THPC implique la réaction de pyrrole et d'aldéhydes aromatiques.

Les composés de l'invention ont fait l'objet d'essais qui ont mis en évidence leurs propriétés thérapeutiques.

A cet effet, les composés de l'invention peuvent être présentés sous forme de médicaments caractérisés en ce qu'ils consistent en un des composés précités ou sous forme de composition pharmaceutique contenant un composé du type précité associé à un excipient.

Les composés de l'invention peuvent être présentés sous toute forme de composition administrable au corps humain ou animal et appropriée à l'administration entérale, parentérale ou transdermique, tel que comprimé, dragée, crème dermique, gélule, capsule, suspension ou solution buvable ou injectable, tel que sirop ou ampoule, timbre transdermique, formulation liposomale (nanoparticules) etc. associés à des excipients convenables et dosés pour permettre une ou plusieurs cures comprenant une ou plusieurs administrations journalières pendant un jour ou plusieurs jours à raison d'une dose comprise par exemple entre 0,1 et 20 mg/kg de poids.

Les formes administrables préférées sont les formes injectables, en particulier sous forme d'une injection intramusculaire ou intraveineuse ou sous forme d'application transcutanée.

Les essais décrits ci-dessous portent sur l'activité de la molécule SIMO1. L'activité de cette nouvelle molécule SIMO1 a été testée in vitro avec des essais de toxicité et de phototoxicité et in vivo par une étude de phototoxicité par évaluation des retards de croissance de tumeurs par rapport à des animaux non traités.

La pharmaco cinétique de SIMO1 a également été étudiée pour déterminer le retard pour une incorporation maximale de STMO1 comparé au retard d'incorporation de la m-THPC (molécule classique largement utilisée dans le domaine de la photo-chimiothérapie). L'analyse de l'élimination de SIM01 par rapport à m-THPC a aussi été étudiée.

La SIMO1 est un dérivé de porphyrine de poids moléculaire 524. La SIMO1 montre, dans une solution isotonique saline, 3 spectres principaux d'absorption à 428, 513 et 652 nm. La solution saline a été obtenue comme suit : un milligramme de SIMO1 a été dissous dans 1 ml de solvant (50 % d'eau, 30 % de PEG et 20 % d'éthanol pur à 99 %). D'autres concentrations ont été obtenues successivement par addition d'une solution isotonique saline.

Pour étudier la toxicité et la phototoxicité de la molécule SIMO1, il a été procédé comme suit :

Des cellules C6 ont été mises en culture dans des flacons falcon de 25 cm² (Polylabo, Strasbourg, France) dans un milieu RPMI supplémenté avec 10 % (V/V) de sérum de veau foetal (FCS), 100 unités de pénicilline mL⁻¹, 100 mg de streptomycine et 2 mM de glutamine.

Les cellules ont été repiquées en culture par dispersion avec 0,025 % de trypsine dans 0,02 % d'acide éthylène-diaminetétraacétique (EDTA) pendant un temps de contact de 2 minutes et remises à adhérer suivant une dilution 1:3, ce qui a maintenu les cellules dans la phase exponentielle de croissance. Les cellules ont été vérifiées sur une base régulière par contamination des mycoplasmes.

Les cellules ainsi cultivées ont été soumises à un traitement photodynamique. Des aliquotes (11 µl) de la solution photosensibilisante SIMO1 ont été ajoutées à des cellules C6 adhérées dans des puits de plaques à 96 puits après trypsination comme décrit ci-dessus. La concentration cellulaire était de 5.10⁴ cellules/ml⁻¹ (100 µl par puits). La concentration finale des solutions de SIMO1 était comprise dans la plage de 0,5 à 50 µg/ml. Immédiatement après addition du photosensibilisant SIMO1, les plaques de cellules ont été maintenues dans une obscurité complète jusqu'au temps de comptage à l'exception d'une irradiation laser des cellules traitées au PDT. Des milieux frais contenant du FCS mais exempt de photosensibilisant ont été préparés avant irradiation laser. Un laser à diode à 650 nm a été utilisé. La puissance à l'extrémité de la fibre a été réglée, en utilisant un dispositif de mesure de puissance (Coherent, France), à 500 mW. La lumière a été transmise à la cible par une fibre optique à une distance de 20 mm afin d'irradier des cellules dans un puits de 6 mm de diamètre à l'intérieur d'un champ unique fournissant une illumination de la totalité de la zone. Le temps d'exposition a été de 13 secondes par puits à 650 nm fournissant une densité d'énergie de 20 J/cm².

Les cellules ont été incubées avec SIMO1 pendant 5 ou 20 heures. Pour les essais de phototoxicité, les cellules ont été lavées et le milieu a été remplacé immédiatement avant irradiation laser.

Les comptages de cellules ont été réalisés 24 heures après la fin des expériences afin d'éviter des sous-estimations des cellules survivantes en écartant des cellules altérées mais vivantes. Au moment du comptage, 15 µl de solution saline tampon au phosphate (solution PBS-MTT, 5 mg/ml⁻¹) ont été ajoutés aux puits. Après 4 heures, 150 µl d'isopropanol-HCl 0,04N ont été ajoutés conformément à la méthode décrite par Mosmann (T. Mosmann. Rapid colorimetric assay for cellular growth and survival : application to proliferation and cytotoxicity assay. J. Immunol. Methods, 65, 55-63, 1985).

La densité optique de chaque puits des microplaques ont ensuite été lues à 570 nm au moyen d'un Uniskan Titerteck (Flow Laboratories, Puteaux, France). Un appareil contenant du RPMI sans rouge de phénol et avec 15 µl d'une solution MTT a été utilisé pour déterminer les blancs pour les mesures d'absorbance.

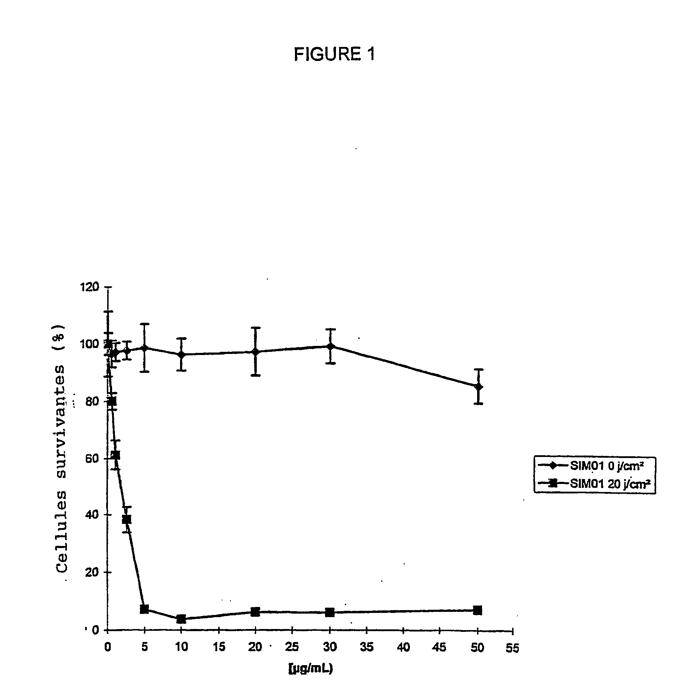

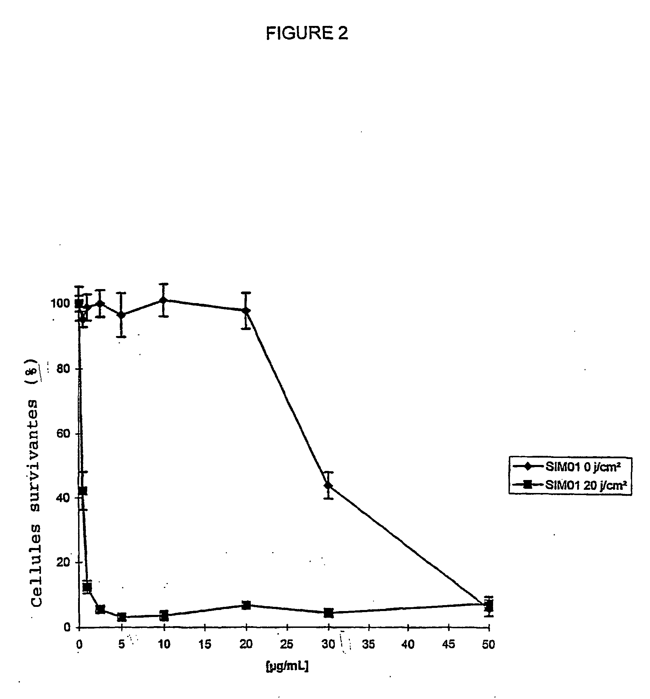

Les résultats sont fournis aux figures 1 et 2 dans lesquelles la figure 1 représente la toxicité et la phototoxicité de SIMO1 sur des cellules C6 laissées à incuber pendant 5 heures avec SIMO1 non irradiées ou irradiées avec un laser à diode à 652 nm à 20 joules/cm² puis traitées au MTT.

La figure 2 se distingue de la figure 1 par le temps d'incubation des cellules C6 avec SIMO1 fixé à 20 heures. Au travers de ces figures, la molécule SIMO1 montre une activité importante in vitro avec une bonne efficacité à de faibles concentrations. Pour le traitement phototoxique (20 joules/cm²), après un temps d'incubation de 5 heures, la DL₅₀ était de 1,75 µg/ml et après 20 heures d'incubation de 0,45 µg/ml. Concernant la toxicité dé SIMO1, aucune toxicité n'a été détectée après 5 heures d'incubation et après 20 heures d'incubation, la DL₅₀ était de 29 µg/ml. Ces tests montrent clairement l'innocuité de SIMO1 en l'absence d'irradiation lumineuse en deçà d'une concentration proche de 20 µg/ml et, à l'inverse, sa phototoxicité certaine dès irradiation.

Afin de confirmer l'utilisation potentielle des composés précités pour la fabrication d'une composition photosensible administrable à un être vivant et utile comme agent apte à engendrer, sous l'effet d'une irradiation, une cytolyse ou une nécrose au moins partielle d'au moins une zone cible du corps humain ou animal, il a été procédé à une visualisation de la localisation in vitro de la molécule SIMO1, ainsi qu'à la détermination de la pharmacocinétique comparée à m-THPC, par imagerie de fluorescence afin de confirmer que les composés précités étaient aptes à pénétrer dans les cellules. Ainsi, des cellules C6 ont été ensemencées à 10⁵ cellules/ml, sur des lames de verre circulaire après trypsinisation. Après 24 heures, des cellules ont été incubées avec SIMO1 (10 µg/ml) à 37°C pendant 3 heures et ensuite lavées dans du PBS (pH 7,2). L'analyse de la fluorescence de SIMO1 (émettant à 650 nm) a été réalisée après excitation de 450 nm à 480 nm par une lampe Xénon 150 W en utilisant une caméra vidéo noir et blanc à détection de photons très sensible (Kappa CF 8/4; Fischer Scientific S. A., France) reliée à un microscope optique (Olympus BX 40, France) équipé avec un objectif à immersion d'huile grossissant 100 fois.

L'intensité de fluorescence la plus forte a été localisée principalement dans le cytoplasme des cellules C6. Les images produites immédiatement après incubation pendant 3 heures avec SIMO1 démontrent clairement que les composés sont aptes à pénétrer dans les cellules.

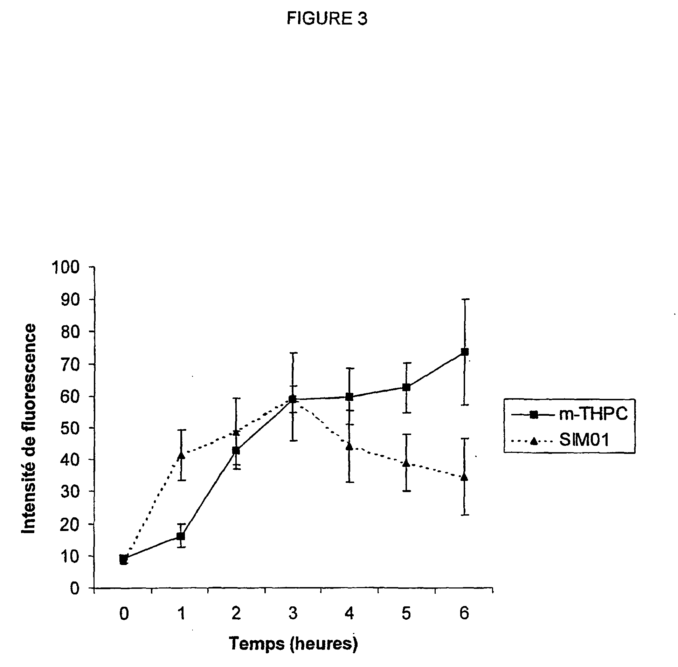

Concernant la pharmacocinétique (Figure 3), la concentration maximale de SIM01 a été observée 3 heures après incubation, avec une forte incorporation dés une heure, puis un diminution de l'intensité de fluorescence a été observée de 3 heures à 6 heures. Pour m-THPC, la fluorescence intracellulaire augmente jusqu'à 6 heures, avec une faible incorporation de photosensibilisant 1 heure après incubation. L'incorporation ainsi que l'élimination plus rapide de SIM01 est due au caractère plus hydrophiles de la molécule, celle-ci ne possédant que deux phényles autour du noyau tétrapyrollique central comparé à m-THPC qui en possède quatre.

Les tests in vitro ont été suivis d'essais toxiques et phototoxiques in vivo afin de démontrer l'intérêt des composés précités notamment sous forme de composition utile à des fins de diagnostic ou de traitement en démontrant que ces compositions, qui comprennent au moins un agent photosensible, sont aptes à induire in vivo lorsqu'elles sont soumises à une irradiation lumineuse et à une longueur d'onde déterminée une nécrose ou une cytolyse au moins partielle d'au moins une zone cible du corps humain ou animal, cette nécrose ou cytolyse étant mesurée notamment en terme de retard de croissance d'une tumeur par rapport à une tumeur témoin. Ces essais ont permis de déterminer d'une part la période de temps optimale devant être laissée entre l'injection et l'irradiation au laser, d'autre part l'efficacité de la molécule SIMO1 par comparaison avec des molécules connues, en particulier le m-THPC qui est un agent photosensible dérivé d'une chlorine.

Pour permettre ces conclusions, il a été procédé tel que suit : des souris Nude/Nude suisses mâles âgées de 7 à 9 semaines (plage de poids 25-35 g) ont été obtenues à partir de Iffa-Credo (L'arbresle, France).

La méthode de test est celle de la tumeur cellulaire HT29 obtenue initialement à partir d'adénocarcinome colorectal humain.

Des greffons de tumeur ont été obtenus comme suit : des tissus non nécrosés de tumeurs solides (diamètre 1 à 2 cm) ont été enlevés immédiatement après mort de la souris donneuse et ensuite mécaniquement broyés dans 1 ml d'une solution saline à 0,9 %. Cette solution (0,2 ml) a été injectée de manière sous-cutanée dans la patte arrière de chaque souris. Cette tumeur a été utilisée une semaine plus tard, quand son diamètre était de 18-20 mm. SIMO1 a été injecté intrapérinéalement dans une solution saline et 12 heures plus tard la souris a été anesthésiée et la tumeur exposée à une lumière de 300 J/cm⁻². La longueur d'onde pour l'éclairage a été choisie entre 652-653 nm.

La source lumineuse était un laser à diode à 652 nm.

La densité lumineuse a été maintenue au-dessous de 0,3 W.cm⁻², à des doses où les effets thermiques sont indétectables. Une irradiation unique a été réalisée et le temps d'exposition a été calculé en tant que fonction du diamètre de la tumeur pour obtenir une densité d'énergie de 300 J/cm⁻² à une densité lumineuse déterminée constante.

Chaque jour, l'indice de croissance de tumeur a été mesuré en utilisant un pied à coulisse, les diamètres orthogonaux principaux étant ajoutés et divisés par un facteur de 2.

Une comparaison statistique de l'indice de croissance entre les souris traitées et les souris de contrôle (sans lumière ou SIMO1) a été faite en utilisant le test de Student.

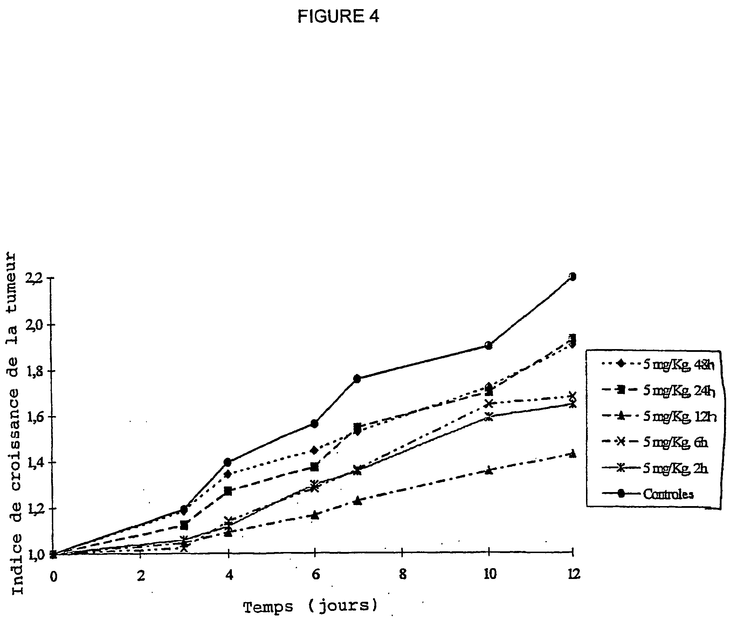

Toutes les conditions de traitement testées comprenant l'agent photosensible et la lumière ont induit une réduction de la croissance de la tumeur comparé au groupe de contrôle non traité. Les effets les plus marquants ont été observés pour un traitement incluant un retard de 12 heures entre une injection intrapéritonéale et une irradiation laser. 12 jours après le traitement, la croissance de la tumeur avait ralenti de 40 %. Ces résultats sont illustrés à la figure 4 dans laquelle la croissance d'une tumeur induite par HT-29 est mesurée après irradiation à 300 J/cm² avec un laser à diode 652 nm 2 heures, 6 heures, 12 heures, 24 heures et 48 heures après injection intrapéritonéale de 5 mg/kg de SIMO1. Un groupe de contrôle n'ayant reçu aucune exposition à la lumière ou aucun agent photosensible a été constitué.

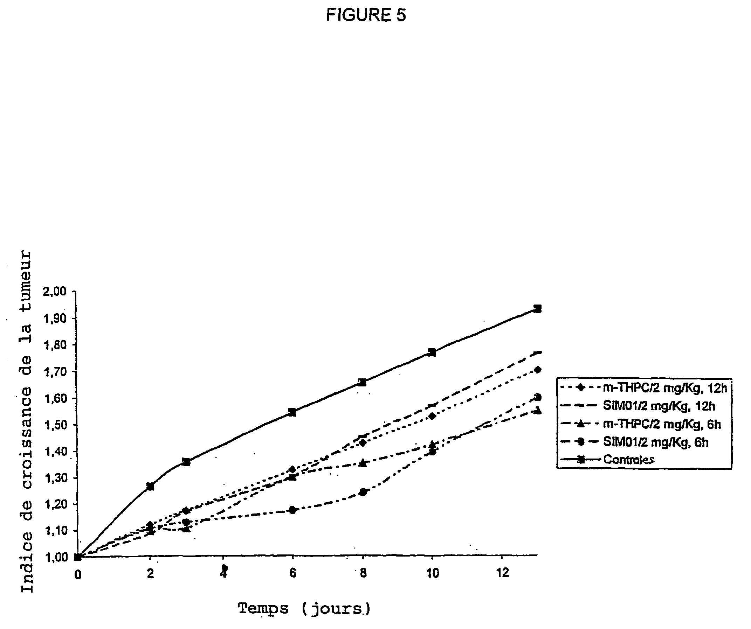

La figure 5 illustre les mêmes expériences que la figure 4 réalisées pour des groupes de souris traitées soit avec SIMO1, soit avec m-THPC et soumises à une irradiation 6 heures et 12 heures après injection intrapéritonéale de 2 mg/kg de SIMO1 ou de m-THPC. On note une activité comparable entre les deux molécules.

De nouveaux tests ont été effectués pour déterminer la période à laquelle on note une incorporation maximale des composés dans les tissus en fonction de la nature des tissus et le temps de rétention du composé par lesdits tissus. Ces essais ont été faits de manière comparative entre la molécule SIMO1 et la molécule m-THPC.

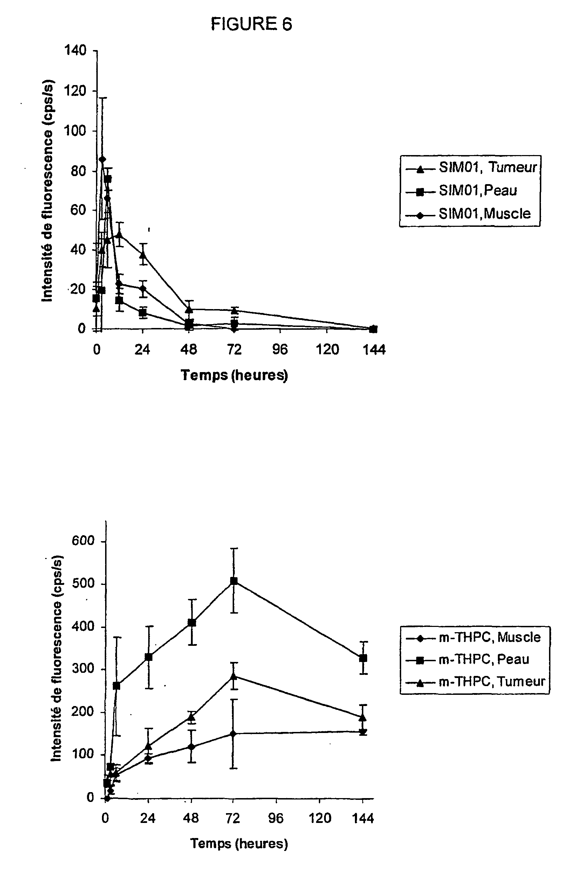

Pour ces essais, il a été procédé comme suit : des mesures spectrofluorimétriques ont été réalisées sur des souris greffées avec HT-29 à différents intervalles de temps après injection de 2 mg/kg⁻¹ de m-THPC ou SIMO1. Les temps testés ont été de 3, 6, 12, 24, 48 et 144 heures sur des souris. Les résultats sont fournis en figure 6.

Les niveaux de fluorescence ont été enregistrés dans différents tissus, à savoir le muscle, la peau et la tumeur. Les mesures ont été déterminées en utilisant une fibre optique placée directement en contact avec les tissus. Au moins quatre spectres par tissu et par souris ont été enregistrés (au moins cinq souris ont été utilisées pour chaque condition expérimentale). Muscle, peau et tumeur ont été successivement étudiés.

Le pic de fluorescence a été observé à 650 nm pour SIM01 et m-THPC. Les spectres d'émission de fluorescence de tissus de souris non traités ont été enregistrés à trois occasions séparées afin d'estimer des variations intersouris. Les valeurs d'intensité de fluorescence mentionnées à la figure 6 correspondent pour chaque organe au résultat de la différence entre la valeur de fluorescence obtenue chez une souris traitée et la valeur de fluorescence enregistrée dans le groupe de contrôle. Les résultats de mesure spectrofluorométrique in vivo ont été exprimés en coups par seconde (unité arbitraire).

Les résultats sont fournis à la figure 6. La molécule SIMO1 montre des temps de rétention plus courts dans tous les tissus comparés à la molécule m-THPC. Le maximum d'incorporation pour SIMO1 est noté entre 6 et 12 heures après injection alors que le maximum pour m-THPC est atteint entre 48 et 72 heures. Pour SIMO1, aucun agent photosensible n'a été détecté après 48 heures alors que pour le m-THPC, l'agent photosensible était encore détectable 144 heures après injection. La molécule SIMO1 montre donc clairement un temps d'incorporation et un temps de rétention plus courts que les agents photosensibles connus à ce jour. Ce temps de rétention plus court permet de réduire le temps d'hospitalisation des patients, de limiter les effets secondaires de tels traitements lourds chez ces derniers et d'éviter des traitements contre les radiations lumineuses puisque le médicament est éliminé rapidement de l'organisme.

Cette cinétique d'incorporation plus rapide associée à une élimination plus rapide par rapport au m-THPC ou d'autres composés, doit être reliée à la structure originale de la molécule objet de l'invention qui ne compte que deux phényles.

Les propriétés spécifiques des composés mentionnés ci-dessus en terme de temps de rétention et de temps d'incorporation dans les tissus à traiter peuvent permettre de supposer l'intérêt desdits composés dans d'autres applications, en particulier en tant que marqueurs pour des dispositifs d'imagerie médicale, ces marqueurs étant caractérisés en ce qu'ils contiennent comme agent de marquage fluorescent au moins l'un des composés précités.

Les composés précités pourront encore être retenus dans le cadre d'application à la fabrication de photopile, au moins l'une des couches constitutives de la photopile étant un composé du type précité.

Bien évidemment, comme il a été mentionné ci-dessus, une composition incorporant de tels composés pourra, dans le cas d'une application à des fins thérapeutiques ou de diagnostics, se présenter sous diverses formes, en particulier sous forme de comprimés, de pilules, de capsules, de granules et de poudres de solution, etc. administrables oralement ou entéralement. Elle pourra également se présenter sous forme d'une préparation injectable pour être administrée parentéralement ou encore sous forme de suppositoires. Elle pourra encore être préparée sous forme d'une préparation topique pouvant se présenter sous forme d'une crème, d'une poudre, d'un liquide ou autre applicable localement sur ladite zone à traiter.

Les doses de cet agent photosensible dépendront des symptômes, de l'âge et du sexe du patient et éventuellement d'autres facteurs, en particulier du type d'agent photosensible utilisé dans le cadre du traitement par photochimiothérapie et de la source de lumière utilisée.

En conclusion, ces composés, qui répondent clairement aujourd'hui aux besoins exprimés dans le domaine de la photochimiothérapie, devraient à l'avenir trouver d'autres applications, notamment dans le domaine de l'imagerie médicale et celui de l'optoélectronique.

## Revendications

1. Composé dérivé de dihydroporphyrine répondant à la formule générale (I) : 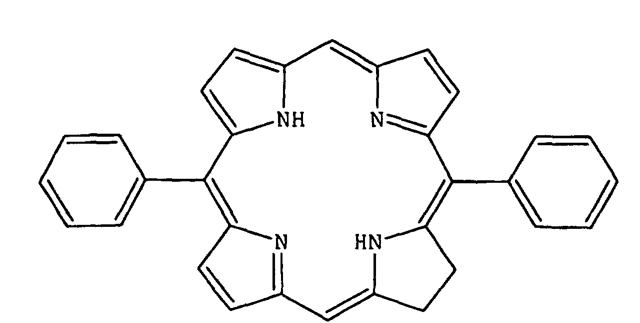 dans laquelle les groupes phényles ne sont pas substitués
ou à la formule générale (II) : 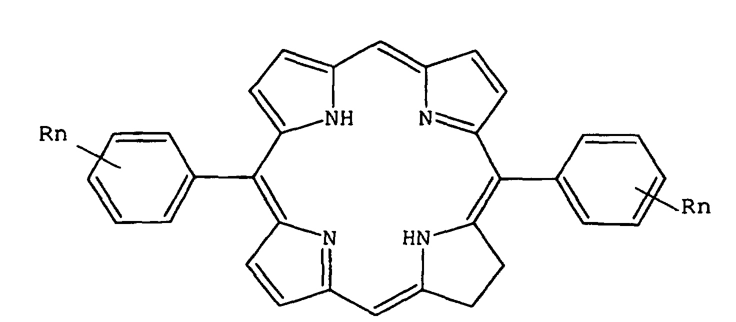 dans laquelle le groupe phényle est substitué, n est égale de 1 à 5, de préférence égale de 1 à 3, et chaque substituant R, qui peut être identique ou différent, et dans des positions identiques ou différentes sur son groupe phényle substitué est un groupe hydroxy (-OH), amine (-NH₂), sulphydrile (-SH), phosphonate (PO₃H₂, PO₃Na₂), éthylphosphonate (PO₃Et₂), sulfonate, alkyl substitué ou non, cycloalkyl substitué ou non, aliphatique, acide aminé, peptide ou polypeptide, pyridine avec différentes positions pour l'atome d'azote, purine, pyrimidine, nucléoside, saccharide, polysaccharide, acide carboxylique, un groupement amide, un groupement ester, un ammonium quaternaire substitué ou non
ou à l'un de leurs sels
ou à l'un de leurs complexes métalliques.

2. Composé dérivé de dihydroporphyrine selon la revendication 1,
**caractérisé en ce qu'**il répond à la formule générale (III):

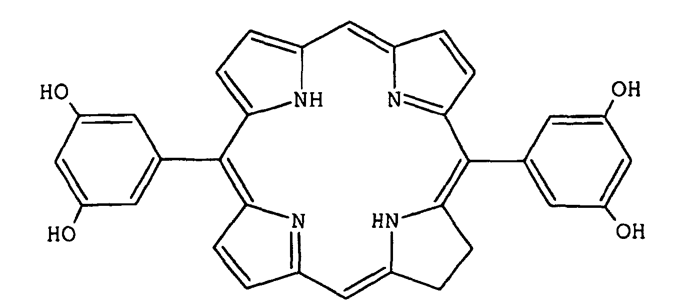

3. Médicament,
**caractérisé en ce qu'**il consiste en un composé selon l'une des revendications 1 et 2.

4. Composition pharmaceutique,
**caractérisée en ce qu'**elle contient un composé selon l'une des revendications 1 et 2 associé à un excipient.

5. Composition utile à des fins de diagnostic ou de traitement du type comprenant au moins un agent photosensible apte à induire in vitro ou in vivo lorsqu'il est soumis à une irradiation lumineuse à une longueur d'onde déterminée, la nécrose ou la cytolyse au moins partielle d'au moins une zone cible du corps humain ou animal,
**caractérisée en ce que** ledit agent photosensible est un composé selon l'une des revendications 1 et 2.

6. Composition selon la revendication 5
**caractérisée en ce qu'**elle se présente sous une forme administrable au corps humain ou animal par voie digestive ou parentérale, en particulier par injection intramusculaire ou intraveineuse ou par application transcutanée.

7. Composition selon l'une des revendications 5 et 6,
**caractérisée en ce qu'**elle est administrée à une dose comprise dans la plage [0,1 mg/kg-20 mg/kg].

8. Marqueur pour dispositif d'imagerie médicale,
**caractérisé en ce qu'**il contient comme agent de marquage fluorescent au moins un composé conforme à l'une des revendications 1 et 2.

9. Photopile,
**caractérisée en ce qu'**au moins l'une des couches constitutives de la photopile est un composé selon l'une des revendications 1 et 2.

10. Utilisation des composés selon l'une des revendications 1 et 2 pour la fabrication d'une composition photosensible administrable à un être vivant et utile comme agent apte à engendrer, sous l'effet d'une irradiation lumineuse, une cytolyse ou une nécrose au moins partielle d'au moins une zone cible du corps humain ou animal.

## Patentansprüche

1. Aus Dihydroporphyrin abgeleitete Verbindung entsprechend der allgemeinen Formel (I): 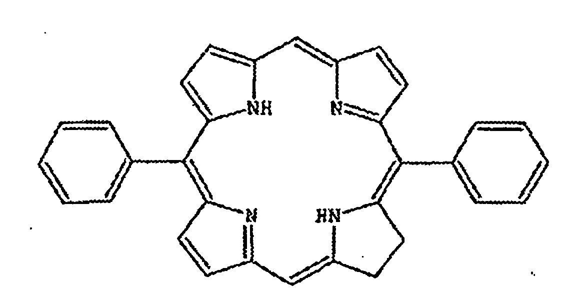 bei der die Phenylgruppen nicht substituiert sind, oder entsprechend der allgemeinen Formel (II): 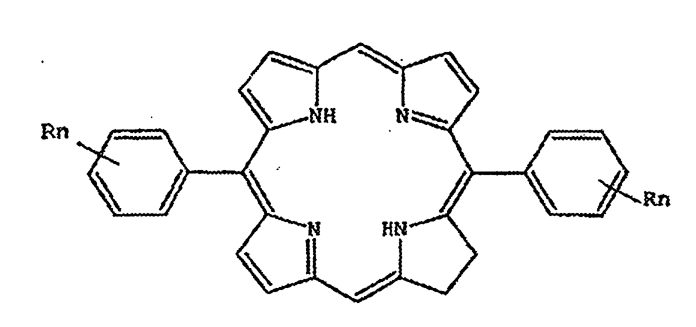 bei der die Phenylgruppe substituiert ist, wobei n gleich 1 bis 5 und vorzugsweise gleich 1 bis 3 ist und jeder Substituent R, welcher identisch oder verschieden und in identischen oder verschiedenen Positionen in seiner substituierten Phenylgruppe angeordnet sein kann, eine Hydroxygruppe (-OH), eine Amingruppe (-NH₂), eine Sulfhydrilgruppe (-SH), eine Phosphonatgruppe (PO₃H₂, PO₃Na₂), eine Ethylphosphonatgruppe (PO₃Et₂), eine Sulfonatgruppe, eine substituierte oder nicht substituierte Alkylgruppe, eine substituierte oder nicht substituierte Cycloalkylgruppe, eine aliphatische Gruppe, eine Aminsäure-, Peptid- oder Polypeptidgruppe, eine Pyridingruppe mit verschiedenen Positionen für das Stickstoffatom, eine Puringruppe, eine Pyrimidingruppe, eine Nucleosid-Gruppe, eine Saccharidgruppe, eine Polysaccharidgruppe, eine Carboxylsäure-Gruppe, eine Amidgruppe, eine Estergruppe, ein substituiertes oder nicht substituiertes quaternäres Ammonium ist, oder entsprechend einem deren Salze oder einem deren Metallkomplexe.

2. Aus Dihydroporphyrin abgeleitete Verbindung nach Anspruch 1, **dadurch gekennzeichnet, dass** sie der allgemeinen Formel (III) entspricht:

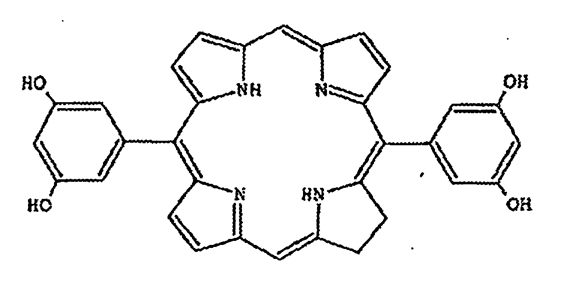

3. Medikament, **dadurch gekennzeichnet, dass** es aus einer Verbindung gemäß einem der Ansprüche 1 und 2 besteht.

4. Pharmazeutische Zusammensetzung, **dadurch gekennzeichnet, dass** sie eine Verbindung nach einem der Ansprüche 1 und 2 enthält, welche mit einem Exzipienten verbunden ist.

5. Zusammensetzung für diagnostische oder therapeutische Zwecke mit mindestens einem lichtempfindlichen Agens, welches geeignet ist, wenn es einer Lichtbestrahlung mit einer bestimmten Wellenlänge ausgesetzt ist, in vitro oder in vivo die zumindest partielle Nekrose oder Cytolyse mindestens eines Zielbereichs des menschlichen oder des tierischen Körpers zu bewirken, **dadurch gekennzeichnet, dass** das besagte lichtempfindliche Agens eine Verbindung nach einem der Ansprüche 1 und 2 ist.

6. Zusammensetzung nach Anspruch 5, **dadurch gekennzeichnet, dass** sie sich in einer Form darstellt, die dem menschlichen oder dem tierischen Körper über den Verdauungsweg oder parenteral, insbesondere durch intramuskuläre oder intravenöse Injektion oder durch transkutane Applikation verabreichbar ist.

7. Zusammensetzung nach einem der Ansprüche 5 und 6, **dadurch gekennzeichnet, dass** sie in einer Dosis im Bereich [0,1 mg/kg - 20 mg/kg] verabreicht wird.

8. Marker für eine medizinische Bildgebungsvorrichtung, **dadurch gekennzeichnet, dass** er als Agens zur fluoreszierenden Markierung mindestens eine Verbindung gemäß einem der Ansprüche 1 und 2 enthält.

9. Fotoelement, **dadurch gekennzeichnet, dass** mindestens eine der das Fotoelement bildenden Schichten eine Verbindung gemäß einem der Ansprüche 1 und 2 ist.

10. Verwendung der Verbindungen gemäß einem der Ansprüche 1 und 2 für die Herstellung einer lichtempfindlichen Zusammensetzung, welche einem Lebewesen verabreichbar und als Agens verwendbar ist, welches geeignet ist, unter der Einwirkung einer Lichtbestrahlung eine zumindest partielle Cytolyse oder Nekrose mindestens eines Zielbereichs des menschlichen oder des tierischen Körpers hervorzurufen.

## Claims

1. Compound derived from dihydroporphyrin corresponding to the general formula (I): 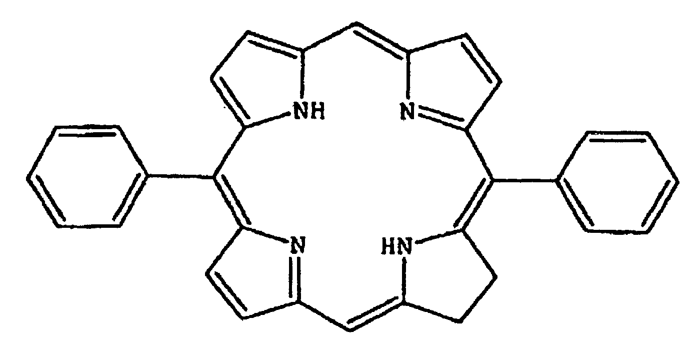 in which the phenyl groups are not substituted, or to the general formula (II): 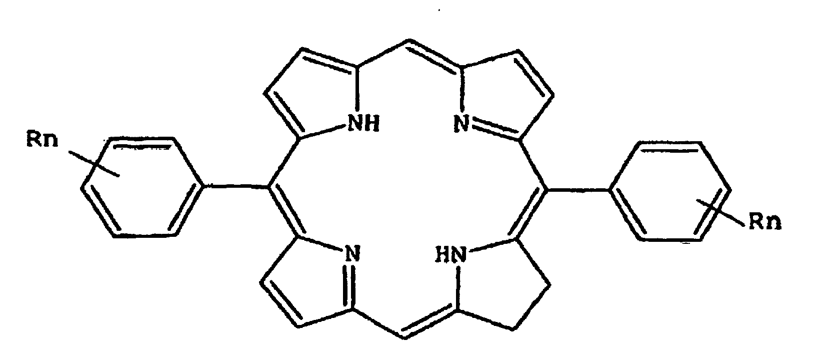 in which the phenyl group is substituted, n is equal to 1 to 5, preferably equal to 1 to 3, and each substituent R, which may be identical or different and in identical or different positions on its substituted phenyl group, is a hydroxyl (-OH), amine (-NH₂), sulphydryl (-SH), phosphonate (PO₃H₂, PO₃Na₂), ethylphosphonate (PO₃Et₂), sulphonate, substituted or unsubstituted alkyl, substituted or unsubstituted cycloalkyl, aliphatic, amino acid, peptide or polypeptide, pyridine, with different positions for the nitrogen atom, purine, pyrimidine, nucleoside, saccharide, polysaccharide or carboxylic acid group, an amide group, an ester group, a substituted or unsubstituted quaternary ammonium,
or to one of the salts thereof
or to one of the metal complexes thereof.

2. Compound derived from dihydroporphyrin according to Claim 1,
**characterised in that** it corresponds to the general formula (III) :

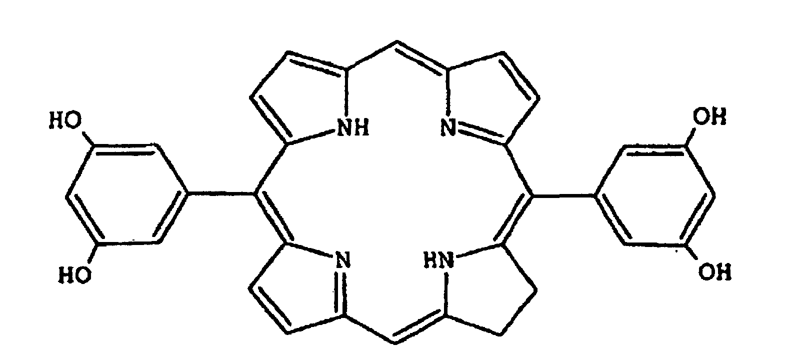

3. Medicament,
**characterised in that** it consists of a compound according to one of Claims 1 and 2.

4. Pharmaceutical composition,
**characterised in that** it contains a compound according to one of Claims 1 and 2 associated with an excipient.

5. Composition useful for diagnostic or treatment purposes, of the type comprising at least one photosensitive agent that, when subjected to irradiation by light of a determined wavelength, is suitable for inducing, in vitro or in vivo, the at least partial necrosis or cytolysis of at least one target zone in the human or animal body,
**characterised in that** the said photosensitive agent is a compound according to one of Claims 1 and 2.

6. Composition according to Claim 5,
**characterised in that** it is in a form in which it can be administered to the human or animal body by digestive or parenteral route, in particular by intramuscular or intravenous injection or by transcutaneous application.

7. Composition according to one of Claims 5 and 6,
**characterised in that** it is administered in a dose in the range [0.1 mg/kg - 20 mg/kg].

8. Marker for medical imaging device,
**characterised in that** it contains at least one compound according to one of Claims 1 and 2 as fluorescent labelling agent.

9. Photoelectric cell,
**characterised in that** at least one of the layers making up the photoelectric cell is a compound according to one of Claims 1 and 2.

10. Use of the compounds according to one of Claims 1 and 2 for the production of a photosensitive composition that can be administered to a living being and is useful as an agent suitable for producing, under the effect of irradiation with light, an at least partial cytolysis or necrosis of at least one target zone in the human or animal body.
